# EUROPEAN PATENT SPECIFICATION

(11) **EP 0 745 243 B1**
(45) Date of publication and mention of the grant of the patent: **10.12.2003**
(21) Application number: 95908098.7
(22) Date of filing: 23.01.1995
(51) Int. Cl.: G06F 15/18, G01N 33/574, G01N 15/14

(54) **AUTOMATED CYTOLOGICAL SPECIMEN CLASSIFICATION METHODS**
AUTOMATISIERTES CYTOLOGISCHES PROBENKLASSIFIZIERUNGSVERFAHREN
PROCEDES DE CLASSIFICATION AUTOMATIQUE DE PRELEVEMENTS CYTOLOGIQUES

(30) Priority: 14.02.1994 US 196714
(43) Date of publication of application: 04.12.1996
(73) Proprietor: AutoCyte North Carolina LLC, Burlington, North Carolina 27215 (US)
(72) Inventor: RUTENBERG, Mark, R., Monsey, NY 10952 (US); RUTENBERG, Akiva, Monsey, NY 10952 (US); KOK, Lanbrecht, Piet, NL-9304 TN Lieveren (NL); BOON, Mathilde, Elisabeth, NL-9304 TN Lieveren (NL); MANGO, Laurie, J., Roosevelt Island, NY 10044 (US)
(74) Representative: Cotter, Ivan John
(86) International application number: US9500853
(87) International publication number: WO95022749

(56) References cited:
- WO-A-91/15826
- US-A- 4 965 725
- US-A- 5 086 476
- US-A- 5 235 522
- US-A- 5 257 182
- MDDI REPORTS GRAY SHEET, issued 23 September 1991, "NeoPath AutoPap 300 Designed to Analyze Pap Smears without Cytologist", page 16.
- ANALYTICAL AND QUATITATIVE CYTOLOGY AND HISTOLOGY, Volume 13, No. 1, issued February 1991, M.S. WACHTEL et al., "Automated Analysis of Rat Breast Tumors", pages 69-73.
- ACTA CYTOLOGICA: THE JOURNAL OF CLINICAL CYTOLOGY AND CYTOPATHOLOGY, Volume 32, No. 2, issued 04 April 1988, F.M. GAVIN et al., "Morphometric Differences Between Cytologically Benign and Malignant Serous Effusions", pages 175-182.
- PROCEEDINGS OF THE NINTH ANNUAL CONFERENCE OF THE ENGINEERING IN MEDICINE AND BIOLOGICAL SOCIETY, issued 04 December 1987, D. TIEN et al., "Automated Cervical Smear Classification", pages 1-2.
- IEEE TRANSACTIONS ON BIOMEDICAL ENGINEERING, Volume 41, No. 9, issued 22 September 1994, F. ERCAL et al., "Neural Network Diagnosis of Malignant Melanoma from Color Images", pages 837-645.

## Description

This application is a continuation-in-part of U.S. Patent No. 4,965,725 and U.S. patent application Serial Nos. 420,105; 425,665, and 502,611 filed October 11, 1989, October 23, 1989, and March 30, 1990, respectively, entitled Automated Cytological Specimen Classification System And Method, the entire disclosures of which are hereby incorporated by reference.

### TECHNICAL FIELD OF THE INVENTION

This invention relates generally to cell classification, particularly to cytology, and, more particularly, to a semi-automated method and apparatus for quickly and accurately classifying cells based on cellular morphology. This invention also relates to use of and the combining of the aforesaid classifying technique and results with immunochemical staining techniques to obtain a quantifiable index or number value for use in diagnostics or other purposes.

### BACKGROUND OF THE INVENTION

In the medical industry there is often the need for an experienced laboratory technician to review a specimen of biological matter for the presence of cells of a certain cellular type. An example of this is the need to review a Pap smear slide for the presence of malignant or premalignant cells. A Pap smear often contains as many as 100,000 to 200,000 or more cells and other objects, each of which a technician must individually inspect in order to determine the possible presence of very few malignant or premalignant cells. Pap smear tests, as well as other tests requiring equally exhausting cell inspection techniques, have therefore suffered from a high false negative rate due to the tedium and fatigue imposed upon the technician.

Several thousand women die each year in the United States alone from cervical cancer; a cancer from which a woman theoretically has a high probability of survival if detected in its early *in situ* stages. If not detected early, however, the chances of survival may decrease. If a malignant cell in a Pap smear is missed, by the time the woman has another Pap smear performed the cancer may have advanced. Consequently, the importance of detecting the presence of only one or a few malignant or premalignant cells among the hundreds of thousands of cells in a smear cannot be overstated. Unfortunately, present manual screening methods are inaccurate. In fact, recently some laboratories have been found to have incorrectly classified as benign up to 30% of the specimens containing malignant or premalignant cells. Also unfortunate is that many prior attempts to automate the cell inspection or classification have been unsuccessful.

Predominately, these prior attempts at automation have relied on feature extraction, template matching and other statistical or algorithmic methods alone. These attempts have required expensive and time-consuming cell preparations to distribute the cells and other objects over a slide so that none of the cells or objects overlap. However, even then these attempts have been unsuccessful at accurately classifying specimens in a reasonable time frame.

These difficulties have been overcome by combining an algorithmic or statistical primary classifier with a neural network based secondary classifier as disclosed in U.S. Patent Application Nos. US-A-4,965,725 and US-A-5,257,182. A commercially available automated Pap smear screener, using a primary classifier in conjunction with a neurocomputer based secondary classifier is produced by Neuromedical Systems, Inc.® of Suffern, New York under trademark PAPNET™.

The use of immunochemical staining techniques in cytology and in histology fields is known. However, in the past there have not been satisfactory techniques for quantifying the examination results of an immunochemical stained specimen. Also, there is no automated or semi-automated analytic technique available for diagnostics assessment of immunocytochemical specimens or immunohistochemical specimens.

### SUMMARY OF THE INVENTION

The present invention provides a method and apparatus for semi-automating a cell classification process using at least primary and secondary classifications followed by review by a skilled technician. Specifically, the process analyzes a specimen which has been stained, such as with an immunochemical marker which indicates information regarding the cell or cells, such as cell proliferation. The specimen is classified and objects within the specimen are ranked according to the likelihood that each represents malignant cells or cell clusters. A display of the highest ranked individual cells and a display of the highest ranked cell clusters is made available for review and manipulation by a cytologist. With the aid of a computer and mouse the cytologist can compute various diagnostically significant information in the displays.

In accordance with an aspect of the invention, a semi-automated method of classifying a specimen for the presence of premalignant or malignant cells, includes the steps of treating a specimen with an immunochemical marker to provide a visual indication of cell proliferation or other biologic characteristic, ranking individual objects in the specimen in an order according to the likelihood that each object has attributes consistent with a premalignant or malignant cell or cell cluster, selecting for display a set of said objects according to said ranking, and displaying images of said selected objects to facilitate review by an operator.

Preferably, the invention includes the step of permitting the operator to select or to eliminate cells, images of one or more cells, sometimes referred to as or included in "tiles", etc., thereby to edit the display to designate or to focus on those cells of particular diagnostic interest. The invention may also include the steps of integrating the stained regions or areas of certain designated displayed cells. Such integration may take the form of classical integration, image processing techniques, counting of number of cells, etc.

The present invention combines morphology with immunochemical staining techniques as applied to cytology; and the invention also may be applied with respect to cell blocks or tissue sections, which also is known as or the same as an histological specimen. Thus, the invention relates to the field of cytopathology and to the field of histopathology. Where description is presented herein concerning cytological samples or specimens, it will be appreciated that the description also will be appropriately applicable to histological samples or specimens which are responsive or susceptible to an appropriate immunochemical stain, such as a MiB-1 stain.

Immunochemical specimens have not been amenable in the past to automated or semi-automated quantitative techniques. Rather, such stains were used to "work up" a specimen. In the past there was no automated or semi-automated correlation made between cytological or histological objects that were stained, e.g., with an immunochemical stain, and cytological or histological objects having morphological abnormality or some other characteristic of interest. In contrast, the present invention relates to obtaining a quantitative index of an immunochemical stain of a cytological specimen or an histological specimen that may correlate clinically, e.g., be a prognostic indicator.

According to an aspect of the invention, a cell classification technique, such as a classification technique of the type currently known as the PAPNET system and technique, such as is described herein and is disclosed in the related patent applications of which this is a continuation-in-part, is used to find or to help find cells of interest, e.g., those for which further diagnostic or medical consideration would be desired or warranted; and an immunochemical staining technique is used to provide information about such cells of interest. Since the stained cells are pre-selected based on the aforesaid classification technique, a quantification of the results of the staining of such cells can be made. Such quantification, then, can be based on the selected cells, rather than on the entire specimen.

The present invention in a sense combines morphology with immuno-stain process analysis of cytological specimens. As a result, it is possible to obtain a quantification of activity, proliferation, or other processes or characteristics of the cytological specimen.

In the past a meaningful quantitative index or value relating to immuno-staining could not be obtained in an automated or semi-automated process. Rather, in the past the positive-staining areas of an entire specimen on a slide could be integrated; but this did provide useful quantification. In the present invention postive-staining cells or cell clusters are selected by the classifier and from those selected a meaningful quantitative index can be generated.

These and other objects, advantages, features and aspects of the present invention will become apparent as the following description proceeds.

To the accomplishments of the foregoing and related ends, the invention, then comprises the features hereinafter fully described in the specification and particularly pointed out in claims, the following description and the annexed drawings setting forth in detail a certain illustrative embodiment of the invention, this being indicative, however, of but one of the various ways in which the principals of the invention may be employed. It will be appreciated that the scope of the invention is to be determined by the claims and the equivalents thereof.

### BRIEF DESCRIPTION OF THE DRAWINGS

In the annexed drawings:
Figure 1 is a schematic illustration of a cytological classification or screening device in accordance with the present invention;
Figure 2 is a diagrammatic illustration of the scanning passes which the screening device performs;
Figure 3 is a schematic illustration of the screening device of Figure 1 with particular emphasis on the processing system; and
Figure 4 is a flow diagram schematically illustrating the flow of image data through the classification device.

### DETAILED DESCRIPTION OF THE INVENTION

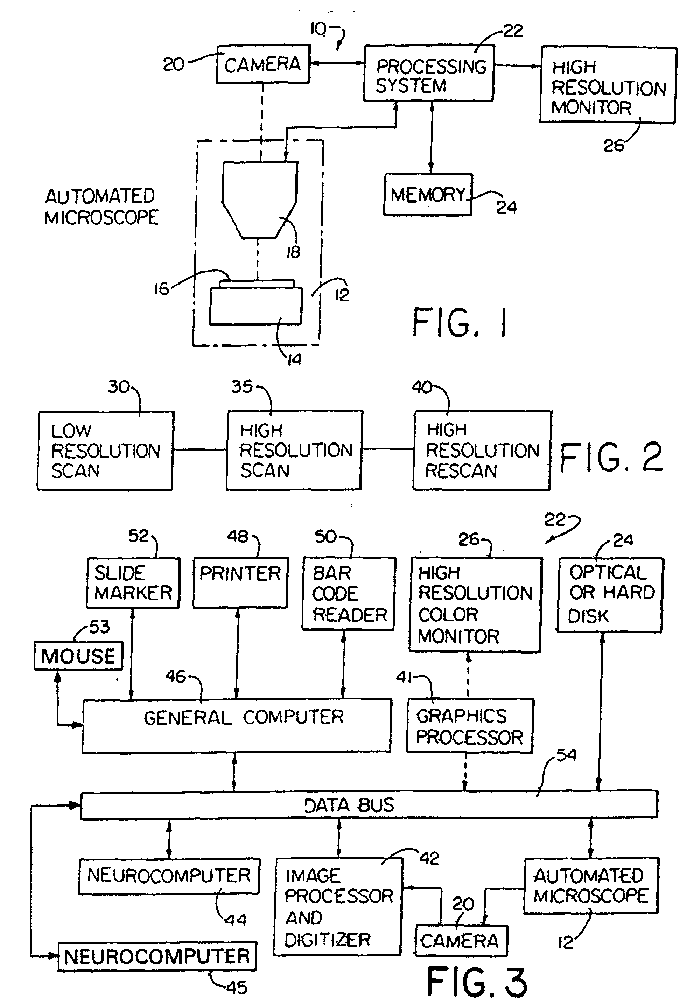

With reference to the several figures in which like reference numerals depict like items, and initially to Figure 1, there is shown a semi-automated cell classification device 10 in accordance with the present invention. Briefly, the device 10 includes an automated optical microscope 12 having a motorized stage 14 for the movement of a slide 16 relative to the viewing region of the viewing portion 18 of the microscope, a camera 20 for obtaining electronic images from the optical microscope, a processing system 22 for classifying objects in the images as likely to be of a predetermined type, and a memory 24 and a high resolution color monitor 26 for the storage and display respectively of objects identified by the processing system as being likely to be of that predetermined type.

In its preferred embodiment the classification device 10 is semi-automated. In other words, the processing system 22 performs a classification of cells and cell clusters in a specimen and ranks the cells and clusters in an order according to the likelihood that each cell and cell cluster is, for example, a malignant or premalignant cell or cell cluster. The cells and cell clusters may be cytological specimens; also, the cell clusters may be a cell block, tissue section, otherwise known as an histological specimen, etc.

The cells and cell clusters which are ranked as most likely to be a premalignant or malignant cell or cell cluster are displayed on the monitor 26 for review by a skilled technician who can then make a final determination regarding whether each displayed cell and cell cluster is premalignant, malignant or otherwise indicative that further medical attention to the patient from whom the specimen was obtained is justified. Preferably the display of cells classified as likely to be malignant, for example, is separate from the display of cell clusters classified as likely to be malignant, such as by presenting the groups of cells or cell clusters one after another on the same monitor 26.

In view of the semi-automated nature of the classification device 10, the microscope 12 will preferably include, in addition to the motorized stage 14, automated apparatus for focussing, for changing lens objectives between high and low power, and for adjustment of the light incident of the slide, as well as circuitry for controlling the movement of the motorized stage, typically in response to a command from the processing system. The microscope may also include an automated slide transport system for moving the slides containing the specimen to be classified on to and off of the motorized stage, a cell dotter for marking relevant areas of the slide, and a bar code reader for reading encoded information from the slide. An example of a microscope performing at least some of these functions is manufactured by Carl Zeiss, Inc. of Germany, and a suitable motorized stage is manufactured by Ludl Electric Products, Ltd. of Hawthorne, New York.

In accordance with the invention the automated microscope 12 preferably performs three scans of the slide having the specimen disposed thereon, as shown diagrammatically in Figure 2. The first scan of the slide is performed at a relatively low magnification, for example 50 power, and is called the low resolution scan (30). The second scan is performed at a higher magnification, for example 200 power, and is called the high resolution scan (35). The third scan is referred to as the high resolution rescan and is also performed at a high magnification (40).

During the first scan (30) of the slide, approximate focal planes for the specific areas of the slide are found and it is determined whether that area of the slide contains a portion of the specimen. Once a low resolution scan (30) has been performed of the whole slide, and the focal planes and areas of the slide containing the specimen have been logged, the high resolution scan (35) is performed.

The high resolution scan (35) is performed only on the areas of the slide found in the low resolution scan (30) to contain a portion of the specimen. Consequently, the comparatively long high resolution scan (35) is performed only on relevant areas of the slide and the processing time is greatly reduced. During the high resolution scan (35), the automated microscope 12 scans the relevant areas of the slide, and the camera 20 takes electronic images of these areas and sends the images to the processing system 22. The processing system 22 performs a primary classification of the image which finds the centroids of biological objects having attributes typical of the cell class for which screening is being performed, such as malignant cells. Using a smaller subimage centered around these centroids, the processing system 22 performs a secondary classification which assigns each centroid a value indicative of the possibility that the object having that centroid is a cell of the type for which classification is being performed. Simultaneously, the centroids are also ranked based on the value assigned through the secondary classification.

Upon completion of the high resolution scan (35), the high resolution rescan (40) is performed for, preferably, the highest ranked sixty-four cells and highest ranked sixty-four cell clusters, although other numbers of cells and cell clusters may be selected by the system. During the rescan (40) the automated microscope 12 will move to each of the highest ranked centroids of the cells and cell clusters and the camera 20 will obtain a high resolution color image of the object having that centroid as well as the contextual image surrounding the object. These high resolution images are then stored in the memory 24 which may be a removable device, such as an optical disk or a tape, etc., or a fixed storage device such as a hard disk. Alternatively, the images may be transferred to another computer via a network or through transportation of the data on a removable storage media.

The sixty-four cell images and sixty-four cell clusters make up two separate summary screens, one for the cell images and one for the cell clusters. The number of images, e.g., 64, is exemplary only; other numbers can be used. The number may be uniform or it may be a function of the ranking, e.g., reflective of the degree of abnormality or interest of the specimen or cell(s) in the specimen. Thus, if there are many cells of interest, the number may be greater than 64; if there are only a few cells of interest, then the number may be less. Each summary screen is preferably arranged as an 8 x 8 matrix of high resolution color images, or tiles, featuring a suspect cell or cell cluster in the center of each image. It will be appreciated, however, that other numbers of images may be displayed concurrently to produce a summary screen, such as a 4 x 4 matrix. These summary screens are displayed on the high resolution color monitor 26 for tertiary analysis and classification, for example, by a cytologist (or if an histological sample, for example, an histologist). This analysis may take place at anytime after the classification process by the processing system 22 has been completed. Further, through the use of a removable memory device or a network connection, the images of the summary screens may be transferred to a work station remote from the microscope 18, camera 20 and processing system 22 for display and analysis. In such an instance a separate graphics processor 41 may be employed to drive the high resolution color monitor 26 and provide a suitable interface with the cytologist.

Herein the screening method and apparatus of the present invention will be described as used in screening a Pap smear for the presence of cervical cancer cells. However, it will be apparent to a person of ordinary skill in the art that this is only an illustrative use and that the present invention may be used in screening samples of other biological matter taken by a variety of cell sampling techniques, such as aspiration and exfoliation to name but two. Further it will be apparent that while the illustrative example screens for malignant or premalignant cells, the screening may be performed for the detection of other cell classes or types. As is mentioned elsewhere herein, the specimen may be a cell block or tissue section, also known in the art as an histological specimen.

Turning now to a more in-depth discussion of the present invention with specific reference to Figure 3, the screening device 10 is shown with particular emphasis on the classification elements embodied in the processing system 22. The processing system 22 preferably includes an image processor and digitizer 42, neurocomputers 44 and 45, and a general processor 46 with peripherals for printing, storage, etc.

The general processor 46 is preferably an Intel® 80486 microprocessor or similar microprocessor based microcomputer although it may be another computer-type device suitable for efficient execution of the functions described herein. The general processor 46 controls the functioning of and the flow of data between components of the device 10, may cause execution of additional primary feature extraction algorithms and handles the storage of image and classification information. The general processor 46 additionally controls peripheral devices such as a printer 48, a storage device 24 such as an optical or magnetic hard disk, a tape drive, etc., as well as other devices such as a bar code reader 50, a slide marker 52, autofocus circuitry, a robotic slide handler, the stage 14, and a mouse 53.

The image processor and digitizer 42 digitizes images from the video camera 20 and performs a primary algorithmic classification on the images to filter out unwanted information. The image processor and digitizer 42 (hereinafter collectively referred to as the image processor) performs two separate filtering operations. In one operation, the images of the specimen taken through the microscope 12 by the video camera 20 are filtered to find images representative of individual cells which have attributes of a premalignant or malignant cell. In a separate operation, which is preferably performed simultaneously with the first filtering operation, cell clusters having attributes of premalignant or malignant cell clusters are found.

Secondary cell classification is performed by the neurocomputers 44 and 45. Each neurocomputer is trained to handle the specific types of images provided by one of the filtering operations of the image processor 42. For example, the neurocomputer 44 is trained to recognize individual premalignant and malignant cells among the output of one of the filtering operations of the image processor 42, while the neurocomputer 45 is trained to recognize premalignant and malignant cell clusters among the images output by the other filtering operation. Alternatively, secondary cell classification functions could be performed using a single neurocomputer, or a template matching algorithm designed to identify shapes known to be typical of a pathological cell or cell cluster. A template matching or other group processing algorithm could be efficiently implemented in a parallel distributed processing network, for example. Another alternative secondary classification embodiment is a holographic image processor designed to perform group based classification.

The image processor 42, the neurocomputers 44 and 45, and the general computer 46 may each access read-only and/or random access memory, as would be readily apparent to one skilled in the art, for the storage and execution of software necessary to perform the functions described relative to that processing component. Further, each component 42, 44, 45, 46 includes circuitry, integrated circuit chips, etc. for the control of communication or data transfer over the data bus 54 as well as other functions typical of similar processors as would be appreciated.

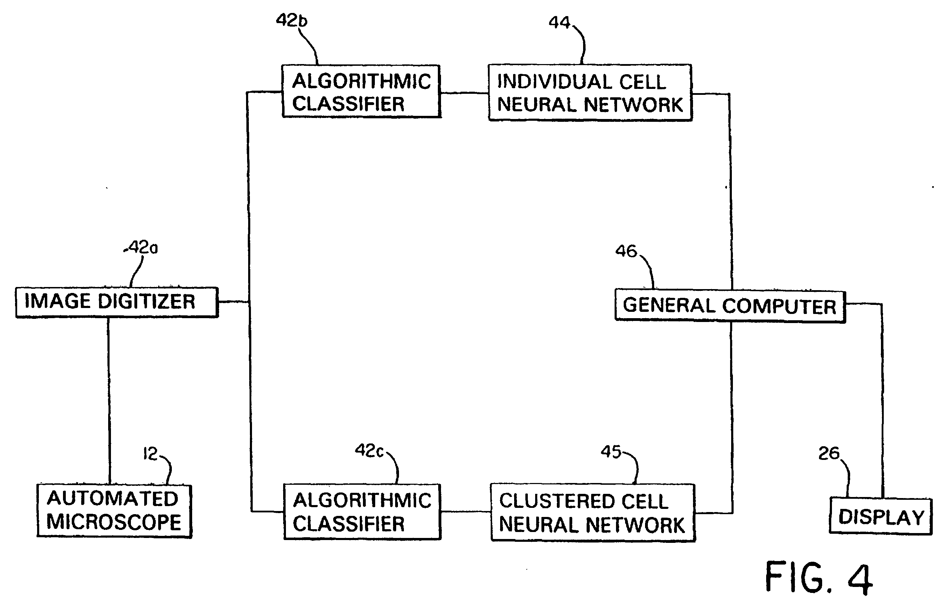

Referring to Figure 4, there is shown a data flow diagram for the processing system 22. As shown, the image digitizer and image processor 42 is divided into three functional elements, 42a, 42b and 42c, to best represent the functional flow of image data through the image processor 42. When the automated microscope 12 focusses on an image in the specimen being analyzed during the high resolution scan (35), the image is presented to the image digitizer 42a which converts the image into a digital format. The digital image data is then subjected to two separate classification processes 42b and 42c designed to perform different filtering operations on the image data. The algorithmic classification functions 42b and 42c perform the primary classification.

The algorithmic classifier 42b is analogous to a band-pass filter which allows individual cells having morphological attributes consistent with a premalignant or malignant cell to pass through the classifier 42b. The classifier 42b filters out other objects and fragments, including cells which are too small to be a malignant cell, such as a neutrophil, cells which are too large, cell clusters, and cells which have other features distinguishing them from malignant cells, for example red blood cells which are distinguishable by their intensity or brightness in the representative image. One embodiment of a suitable morphological algorithm is described in U.S. Patent No. 5,257,182 which is incorporated by this reference. Many types of malignant cells tend to be dissociative and thus the algorithmic classifier 42b is optimized to find these dissociative cells which have the appropriate morphological characteristics of a premalignant or malignant cell and to pass those cells on to the neurocomputer 44 for further classification.

The algorithmic classifier 42c is analogous to a band-pass filter which passes cell groups or clusters and rejects individual, or dissociated, objects and cells. Some types of cancers tend to form cell clusters, for example adenocarcinomas, while other cell clusters, for example, multicellular epithelial fragments and glandular clusters, may have other diagnostic significance to a reviewer, as is discussed more fully below. The algorithmic classifier 42c is optimized to pass images of significant cell clusters to the neurocomputer 45 for further classification.

Preferably the algorithmic classifiers 42b and 42c, as well as the image digitization function 42a, are combined in a single processor 42 which permits both sequential and simultaneous processes. In the preferred embodiment, the image processor and digitizer 42 is a low level morphological feature extraction image classifier which includes among other things an image digitization function and an ISMAP (Iconic to Symbolic Mapping) board. The suitable image processor is described more fully in U.S. Patent No. 4,601,055, the entire disclosure of which is incorporated by this reference. Alternatively, the image processing and digitization functions could be separated into two or more components.

The secondary classification is performed by the neurocomputers 44 and 45. Each neurocomputer 44, 45 is trained to recognize cells or cell clusters, depending upon its training, which are most likely to represent premalignant or malignant cells or cell clusters. The neurocomputer 44 and 45 are provided image data from the algorithmic classifiers 42b, 42c, respectively, which is consistent with its training. For example, the neurocomputer 44 is trained to recognize individual premalignant or malignant cells and is provided with image data which the algorithmic classifier has identified as having the morphological attributes of individual premalignant or malignant cells. Similarly, the neurocomputer 45 is trained to recognize premalignant or malignant cell clusters and is provided with image data which the algorithmic classifier 42c has identified as having the morphological attributes of premalignant or malignant cell clusters.

As noted above, the neurocomputer 44 is provided with image data from the primary algorithmic classifier 42b which consists of images of cells and other objects which the algorithmic classifier has identified as having attributes consistent with dissociated premalignant and malignant cells. In accordance with its training, the neurocomputer 44 assigns images in the data which are likely to represent premalignant or malignant cells with a relatively high value and assigns images that to the neurocomputer are less likely to represent malignant cells, for example benign cells, with a lesser value. The sixty-four highest ranked cell images, i.e., those cell images most likely to represent premalignant or malignant cells are provided to the general computer 46.

The neurocomputer 45 is provided with image data representing cell clusters identified by the primary algorithmic classifier 42c. The neurocomputer 45, in accordance with its training, assigns images in the data which are likely to represent premalignant and malignant cell clusters with a relatively high value while assigning images which are likely to be benign with a lesser value. This time, the highest ranked sixty-four images of cell clusters are provided to the general computer 46.

The neurocomputers 44 and 45 are each preferably a computer embodiment of a neural network trained to identify suspect cells or cell clusters. In this embodiment the parallel structure of a two or three-layer backpropagation neural network is emulated with pipelined serial processing techniques executed on any of a number of commercially available neurocomputer accelerator boards. The operation of these neurocomputers, for example, is discussed in Hecht-Nielsen, Robert, "Neurocomputing: Picking the Human Brain", IEEE Spectrum, March, 1988, pp. 36-41. The neurocomputers are preferably implemented on an Anza Plus™ processor, which is a commercially available neurocomputer of Hecht-Nielsen Neurocomputers. Such a neurocomputer could be easily configured to operate in a manner suitable to perform the secondary classification functions by one of ordinary skill in the art through reference to corresponding manuals, etc.

Preferably each neurocomputer 44, 45 is trained with images which have been classified by the corresponding primary algorithmic classifier and then reviewed by a person skilled in analyzing cytological specimens for the presence of malignant cells and cell clusters. Such a person can readily review the cells and cell clusters which have been classified by the primary algorithmic classifiers 42b and 42c and determine whether the cells or cell clusters are benign or malignant. Operating in a training mode, the neurocomputer 44 is trained with images of individual objects classified as having morphological attributes of a malignant cell by the algorithmic classifier 42b which a skilled technician has determined to be malignant or benign. The images are fed through the neurocomputer and the neurocomputer is informed by the reviewer of the correct classification of the image as benign or malignant. Based on a series of such inputs the neurocomputer can organize itself to associate a known benign image with an output of .1 and a known malignant image with an output of .9. Such outputs represent, for example, the degree of certainty that a cell is normal or abnormal, respectively. When the secondary classifier is presented with new, unknown cells, it generalizes from its training and attaches a net value to the image. The closer that the secondary classifier is able to categorize the unknown image into the benign category, the closer is its net value equal to .1. Conversely, the more closely that the unknown image appears to resemble the nonbenign images of its training set, the closer is the net value assigned to that image equal to .9.

The neurocomputer 45 is trained in the same way but with images of cell clusters which the primary algorithmic classifier 42c has identified as having morphological characteristics of premalignant or malignant cell clusters. These images are further classified by a skilled person who then assigns the images a value between .1 and .9 depending on whether the cell cluster is benign or malignant, and the neurocomputer is provided with the known images and their respective values assigned by the skilled reviewer.

High resolution images of the cells and cell clusters ranked by the neurocomputers 44 and 45 as most likely to be representative of malignant cells or cell clusters are obtained through a rescan (40) of the automated microscope of the relevant areas on the specimen. The general computer 46 then stores the image in the memory 24, which may include an optical disk or tape, and makes the images available for viewing on the monitor 26 in separate summary screens or pages of information or for some other use. As discussed above, the first summary screen is preferably a display of the sixty-four cells classified by the neurocomputer 44 as most likely to be malignant cells. These cells and surrounding material on the slide, called tiles, are displayed in an arrangement, such as an eight by eight matrix, which facilitates review by a technician. A cytologist can easily visually scan this summary screen in search of images of cells having the attributes for which classification is being performed. If the system is being used to screen a Pap smear for the presence of cervical cancer, the cytologist would typically examine visually the first screen for cells having attributes of a malignant or premalignant cervical cell, such as a comparatively large, dark nucleus. The cytologist is preferably permitted to interact with the general computer 46 through a mouse 53 to identify certain cells as malignant, to make other determinations or to cause the general computer to execute other commands or display different material.

The second screen, which includes images of cell clusters, is typically examined for grouped glandular cells, endocervical cells, endometrial cells, and cell groupings peculiar to certain types of cancers, for example, adenocarcinomas. The presence of adenocarcinoma or any other malignant cells or cell clusters is of course direct evidence of cancer. However, other material on the second summary screen may also be of relevance to the cytologist. For example, the presence of endocervical cells facilitates a determination that the specimen contains an adequate sampling of the transitional zone within the cervix where evidence of cancer is most likely to occur in the earlier stages of cancer. Further, the presence of endometrial cells in certain patients, such as post menopausal patients, may also indicate the presence of abnormal conditions including cancer. Manipulation of the screen display as well as interaction with the general computer 46 is again permitted through use of a mouse 53 or other interactive devices including a keyboard.

The Pap smear specimens used herein as an illustrative specimen are obtained by staining a cervical smear with Papanicolaou stain or a similar stain for highlighting the nuclei of the biological matter by staining the nuclei a purple color so that they are easily discernible from the surrounding cytoplasm. Other stains, such as immunolabeling stains, are specific to certain activity or occurrences within a cell and can detect, for example, cell mitosis or division. One such stain is MiB-1 which is a monoclonal antibody to certain antigens expressed in a nucleus of a cell around the time of mitosis. Consequently, the MiB-1 stain can be used to provide a visual indication of cell proliferation. As it is known that malignant cells tend to proliferate at a much faster rate than benign cells, an indication of a region in a specimen undergoing significant proliferation can provide important diagnostic information to the person reviewing the sample. This is especially true in multicellular fragments or clusters where the occurrence of proliferation is high.

The MiB-1 stain effectively dyes the nucleus of a cell brown while not significantly coloring the cytoplasm. In the instance of a Pap smear specimen the MiB-1 stain is useful in providing diagnostic information on the specimen by itself or in conjunction with conventional Pap smear staining. When used in connection with a conventional Pap smear staining, the stained Pap smear is destained using known techniques and then restained with the MiB-1 stain in a procedure such as that described below. When it is not necessary that the specimen be first screened with a Pap smear stain or when a different type specimen is used for which screening with a conventional stain is not desired, the MiB-1 stain may be applied to the specimen following a procedure such as that below without first destaining the specimen.

A suitable procedure for applying the MiB-1 stain to a previously unstained or destained specimen includes retrieving the antigens in the specimen such as by:
1) placing the specimen slide in plastic jar containing 10mM citrate buffer, pH 6.0;
2) placing the plastic jar containing the specimen and citrate buffer in a microwave oven;
3) microwave heating the specimen to 100 degrees Celsius and maintaining the specimen at 100 degree Celsius for approximately 20 minutes;
4) cooling the specimen in the citrate buffer below a maximum temperature of 50 degrees Celsius; and
5) rinsing the specimen in TBS for approximately 2 minutes.

Once the antigen retrieval procedure is completed, the specimen is stained with the MiB-1 stain such as through the standard SAB protocol using an antisera dilution of 1:200 and counter staining with Haematoxylon. The standard SAB protocol is discussed in detail in Kok & Boon, "Microwave Cookbook for Microscopists: Art and Science of Visualization," Third edition, Coulomb Press Leyden, Leiden (1992).

Subjecting a MiB-1 stained specimen to the classification device 10 described above will result in the system producing two summary screen of objects for review by a cytologist. The first summary screen again contains images of individual objects and their contextual surrounding matter that the classification system ranked as most likely to represent premalignant or malignant cells that also stain positively for the immunochemical stain. The second screen contains cell clusters, such as multicellular epithelial fragments which the classification system ranked as most likely to represent premalignant or malignant cell clusters that also stain positively for the immunochemical stain. As a result of the MiB-1 staining, and the selectivity of the stain to proliferating cells and their expressed proteins, the summary screens will contain individual cells and cell clusters which may be undergoing, preparing for or having completed mitosis as well as a number of cells that stain positive but are not malignant or involved in malignant processes, for example, Herpes Virocytes, cells from lymph follicles (follicular cervicitis) and cells from tissue repair. A cytologist can readily distinguish the premalignant and malignant cells and cell clusters from other material and benign processes which are not of interest.

The result of subjecting the MiB-1 stained specimen to the classification device 10 is that not only are morphologically significant cells detected, but also those bearing evidence of proliferation. Consequently, the cytologist examining the specimen can determine the presence of malignant cells and the degree of proliferation of malignant cells. Such information allows a doctor to determine how aggressive of a treatment program is necessary for the patient from whom the specimen was obtained.

In some instances the immunostaining may increase the sensitivity of the neurocomputers 44 and 45 in recognizing premalignant and malignant cells. The neurocomputers 44 and 45 alternatively may be trained with specimens which have been stained with an immunochemical stain.

The cytologist can use the mouse 53 to select cells or cell clusters from the display 20 for grouping on the summary screen with other cells or clusters, to delete cells or clusters from the display as well as to command the general computer 46 to perform certain processing or calculations on the information in the summary screen. For example, a count of the number of proliferating cells in a specimen is a diagnostically significant measurement. The cytologist can review the summary screens, edit the display to remove certain objects or to designate others for counting, and then instruct the general processor 46, such as through use of the mouse 53 or a keyboard, to count the number of proliferating cells in the display. The cytologist can also mark cells in a tile of the summary screen, for example individual cells in an epithelial cell fragment, as positive-staining or negative staining and instruct the general computer 46 to determine the percentage of positive-staining nuclei in a tile or fragment. The classification can also be tailored to provide a total count of the brown stained cells in the specimen, and in conjunction with the neurocomputers and/or the cytologist, a relative count of stained benign cells to stained malignant cells.

Another diagnostically significant measurement is the area of the display that is stained. This is particularly relevant for cell clusters, such as epithelial fragments, which are displayed on the second summary screen. Again, using the mouse 53 for example, the cytologist can review the images on the second screen, edit the screen by removing images, selecting images to form a separate screen of for example 16 significant images, and command the-general computer 46 to determine the area of the display that is stained by the MiB-1 stain. The general processor 46 then calculates the stained area, such as a percentage of the total screen area, using conventional algorithmic techniques, and provides the result to the cytologist.

Through the interaction of the cytologist with the general processor 46 through use of a mouse 53 or similar interactive tool, and the ability to edit the images on the screen and identify characteristics of cells in the display or within individual tiles in the display, the technician can instruct the system to provide a large amount of diagnostic information which is specifically tailored to the specimen reviewed and the cellular material found in the specimen.

While the example given here of an immunolabeling dye is MiB-1 other types of immunochemical markers can be used in equivalent ways to indicate the desired characteristics in the specimen, such as proliferation or other characteristics. One supplier of immunochemical labels is ICN Biochemicals although many other suppliers are available. Further, the classification performed by the image processor 42 and neurocomputers 44 and 45 can be tailored to select images for display based on the detection of a feature made detectible by the immunochemical marker.

Another example of the invention includes the classifying of a cytological specimen or an histological specimen. Such classification determines cells or groups of cells of interest for further consideration, e.g., medical or diagnostic consideration. According to the invention, a technique described above using the PAPNET system provides primary and secondary classification steps and includes use of a neurocomputer or neural net in the classification process. The classification method may be based on morphology. The classification methodology may be expanded or extended beyond morphology to include criteria related to the immunochemical staining of the cells or groups of cells in a cytological specimen or histological specimen.

An example of such expanded processing includes using information relating to the amount of stained material in the objects, tiles, cells, or groups of cells, etc. which were selected by the classification process. Such use may include counting the number of stained objects in the tiles that were selected for display. The expanded processing may include integrating the stained regions or area of the selected tiles that were selected for display. Another type of such expanded processing may include the counting of the number of tiles that were selected for display which tiles also include some or at least a predetermined amount or proportion of stained material, stained region or stained area. These are but several of the ways that the cells, areas where selected cells are located, tiles, etc., determined in the classification process, can be processed to quantify the immunochemical staining in conjunction with selected cells or groups of cells of morphological interest. In this expanded processing technique, it is possible that the system determines the quantifiable value automatically by conventional image processing techniques, image analysis techniques and/or area integration algorithm techniques without intervention by an operator.

Another example of such expanded processing involves a semi-automated approach. In this approach an operator views the display and the cells or groups of cells selected in the classification process. The operator then can edit the displayed sample/specimen for determining what is to be further considered for the quantification process. The operator undesignates (de-selects), e.g., by pointing a mouse cursor at or in a particular displayed tile and clicking the mouse button, cells, groups of cells, objects, tiles, etc. which are not to be included in the expended processing of the type described above, such as image processing/analyzing and integration of the stained areas, etc.

An alternate example of such expanded processing involves a modified semi-automated approach similar to that described just above. In this approach an operator views the display and the cells or groups of cells selected in the classification process. The operator designates (selects), e.g., by pointing a mouse cursor at or in a particular displayed tile and clicking the mouse button, cells, groups of cells, objects, tiles, etc. which are to be included in the expanded processing of the type described above, such as integration of the stained areas, etc.

### STATEMENT OF INDUSTRIAL APPLICATION

The present invention is applicable to cell classification in general and is particularly applicable to the classification of cells in a cervical smear.

## Claims

1. A semi-automated method of classifying a specimen for the presence of premalignant or malignant cells, comprising the steps of:
a) treating a specimen with an immunochemical marker to provide a visual indication of cell proliferation or other biologic process;
b) ranking individual objects in the specimen in an order according to the likelihood that each object has attributes consistent with a premalignant or malignant cell or cell cluster;
c) selecting for display a set of said objects according to said ranking; and
d) displaying images of said selected objects to facilitate review by an operator.

2. The method of claim 1, wherein said individual objects include single cells.

3. The method of claim 1, wherein said individual objects include cell clusters.

4. The method of claim 1, wherein selected objects which represent individual cells are displayed separating from selected objects which represent cell clusters.

5. The method of claim 1, further including permitting the operator to edit the display to designate cells within the images of selected objects as of diagnostic interest.

6. The method of claim 1, further including permitting the operator to edit the display to eliminate from the images of selected objects cells not of diagnostic interest.

7. The method of claim 1, including the step of counting specified cells or cell clusters of interest in said display.

8. The method of claim 1, including the step of counting specified positively stained cells or cell clusters of interest in said display.

9. The method of claim 5, including the step of counting cells designated by the operator as of diagnostic interest.

10. The method of claim 5, including the step of counting cells designated by the operator as positively staining.

11. The method of claim 5, including the step of counting cells automatically selected for display as of diagnostic interest.

12. The method of claim 5, including the step of counting cells automatically selected for display as positively staining.

13. The method of claim 1, wherein said immunochemical marker is MiB-1 stain.

14. The method of claim 1, wherein said immunochemical marker stains the nuclei of proliferating cells.

15. The method of claim 14, including the step of calculating the area of said stained nuclei.

16. The method of claim 1, wherein said cells are an histological specimen.

17. A method of classifying a specimen for the presence of premalignant or malignant cells, comprising the steps of:
a) treating a specimen with an immunochemical marker to provide a visual indication of cell proliferation or other biologic process;
b) ranking individual objects in the specimen in an order according to the likelihood that each object has attributes consistent with a premalignant or malignant cell or cell cluster;
c) selecting for a set of said objects according to said ranking; and
d) determining a quantitative index of an immunochemical stain of the specimen.

18. The method of claim 17, said determining comprising determining such quantitative index for correlation clinically as a prognostic indicator.

19. The method of claim 17, comprising selecting the specimen as a cytological or histological specimen.

20. A method of classifying a cytological or histological specimen, comprising the steps of:
a) treating a specimen with an immunochemical marker to provide a visual indication of cell proliferation or other biologic process;
b) ranking individual objects in the specimen in an order according to the likelihood that each object has attributes consistent with a specified interest;
c) selecting for a set of said objects according to said ranking; and
d) determining a quantitative index of an immunochemical stain of the specimen.

## Patentansprüche

1. Halbautomatisiertes Verfahren zur Klassifizierung einer Probe hinsichtlich des Vorhandenseins prämaligner oder maligner Zellen mit den Stufen, in denen man
a) eine Probe zur Bereitstellung einer visuellen Anzeige von Zellproliferation oder eines anderen biologischen Prozesses mit einem immunochemischen Marker behandelt,
b) einzelne Objekte in der Probe in einer Reihenfolge entsprechend der Wahrscheinlichkeit, daß jedes Objekt Eigenschaften aufweist, die mit einer prämalignen oder malignen Zelle oder Zellcluster in Einklang stehen, einordnet,
c) einen Satz der Objekte entsprechend der Einordnung für eine Anzeige auswählt und
d) Abbildungen der ausgewählten Objekte zur Erleichterung der Durchsicht durch einen Anwender anzeigt.

2. Verfahren nach Anspruch 1, wobei die einzelnen Objekte einzelne Zellen umfassen.

3. Verfahren nach Anspruch 1, wobei die einzelnen Objekte Zellcluster umfassen.

4. Verfahren nach Anspruch 1, wobei die ausgewählten Objekte, welche einzelne Zellen repräsentieren, getrennt von ausgewählten Objekten, welche Zellcluster repräsentieren, angezeigt werden.

5. Verfahren nach Anspruch 1, welches weiterhin umfaßt, daß es dem Anwender möglich ist, die Anzeige zur Bestimmung von Zellen innerhalb der Abbildungen ausgewählter Objekte, soweit sie von diagnostischem Interesse sind, zu bearbeiten.

6. Verfahren nach Anspruch 1, welches weiterhin umfaßt, daß es dem Anwender möglich ist, die Anzeige zur Eliminierung von Zellen, die nicht von diagnostischem Interesse sind, von den Abbildungen ausgewählter Objekte zu bearbeiten.

7. Verfahren nach Anspruch 1, welches die Stufe umfaßt, bei der man bestimmte interessierende Zellen oder Zellcluster in der Anzeige zählt.

8. Verfahren nach Anspruch 1, welches die Stufe umfaßt, in der man bestimmte interessierende positiv gefärbte Zellen oder Zellcluster in der Anzeige zählt.

9. Verfahren nach Anspruch 5, welches die Stufe umfaßt, in der man Zellen, die von dem Anwender als von diagnostischem Interesse bestimmt wurden, zählt.

10. Verfahren nach Anspruch 5, welches die Stufe umfaßt, in der man Zellen, die von dem Anwender als positiv färbend bestimmt wurden, zählt.

11. Verfahren nach Anspruch 5, welches die Stufe umfaßt, in der man Zellen, die als von diagnostischem Interesse für eine Anzeige automatisch ausgewählt wurden, zählt.

12. Verfahren nach Anspruch 5, welches die Stufe umfaßt, in der man Zellen, die als positiv färbend für eine Anzeige automatisch ausgewählt wurden, zählt.

13. Verfahren nach Anspruch 1, wobei der immunochemische Marker MiB-1-Färbemittel ist.

14. Verfahren nach Anspruch 1, wobei der immunochemische Marker die Kerne von proliferierenden Zellen färbt.

15. Verfahren nach Anspruch 14, welches die Stufe umfaßt, in der man die Fläche der gefärbten Kerne berechnet.

16. Verfahren nach Anspruch 1, wobei die Zellen eine histologische Probe sind.

17. Verfahren zur Klassifizierung einer Probe hinsichtlich des Vorhandenseins prämaligner oder maligner Zellen mit den Stufen, in denen man
a) eine Probe zur Bereitstellung einer visuellen Anzeige von Zellproliferation oder eines anderen biologischen Prozesses mit einem immunochemischen Marker behandelt,
b) einzelne Objekte in der Probe in einer Reihenfolge entsprechend der Wahrscheinlichkeit, daß jedes Objekt Eigenschaften aufweist, die mit einer prämalignen oder malignen Zelle oder Zellcluster in Einklang stehen, einordnet,
c) einen Satz der Objekte hinsichtlich ihrer Einordnung auswählt und
d) einen quantitativen Index einer immunochemischen Färbung der Probe bestimmt.

18. Verfahren nach Anspruch 17, wobei die Bestimmung umfaßt, daß man den quantitativen Index für eine Korrelation als einen prognostischen Indikator klinisch bestimmt.

19. Verfahren nach Anspruch 17, bei dem man die Probe als eine zytologische oder histologische Probe auswählt.

20. Verfahren zur Klassifizierung einer zytologischen oder histologischen Probe mit den Stufen, in denen man
a) eine Probe zur Bereitstellung einer visuellen Anzeige von Zellproliferation oder eines anderen biologischen Prozesses mit einem immunochemischen Marker behandelt,
b) einzelne Objekte in der Probe in einer Reihenfolge entsprechend der Wahrscheinlichkeit, daß jedes Objekt Eigenschaften hat, die mit einer bestimmten Bedeutung in Einklang stehen, einordnet,
c) einen Satz der Objekte hinsichtlich ihrer Einordnung auswählt und
d) einen quantitativen Index einer immunochemischen Färbung der Probe bestimmt.

## Revendications

1. Procédé de classification semi-automatique d'un prélèvement quant à la présence de cellules prémalignes ou malignes, comprenant les étapes consistant à :
a) traiter un prélèvement avec un marqueur immunochimique pour obtenir une indication visuelle de la prolifération cellulaire ou de tout autre processus biologique ;
b) classer des objets individuels dans le prélèvement selon un ordre en fonction de la probabilité pour chaque objet de posséder des attributs en rapport avec une cellule ou un agrégat de cellules prémalignes ou malignes ;
c) sélectionner, pour l'afficher, un ensemble desdits objets conformément audit classement ; et
d) afficher les images desdits objets sélectionnés pour faciliter la révision par un opérateur.

2. Procédé selon la revendication 1, dans lequel lesdits objets individuels comprennent des cellules uniques.

3. Procédé selon la revendication 1, dans lequel lesdits objets individuels comprennent des agrégats de cellules.

4. Procédé selon la revendication 1, dans lequel les objets sélectionnés qui représentent des cellules individuelles sont affichés séparément des objets sélectionnés qui représentent des agrégats de cellules.

5. Procédé selon la revendication 1, consistant en outre à permettre à l'opérateur d'éditer l'affichage pour désigner, au sein des images d'objets sélectionnés, des cellules comme ayant un intérêt diagnostique.

6. Procédé selon la revendication 1, consistant en outre à permettre à l'opérateur d'éditer l'affichage pour éliminer des images d'objets sélectionnés les cellules qui n'ont pas d'intérêt diagnostique.

7. Procédé selon la revendication 1, comprenant l'étape consistant à compter les cellules ou agrégats de cellules spécifiés d'intérêt dans ledit affichage.

8. Procédé selon la revendication 1, comprenant l'étape consistant à compter les cellules ou agrégats de cellules spécifiés et colorés positivement d'intérêt dans ledit affichage.

9. Procédé selon la revendication 5, comprenant l'étape consistant à compter les cellules désignées par l'opérateur comme ayant un intérêt diagnostique.

10. Procédé selon la revendication 5, comprenant l'étape consistant à compter les cellules désignées par l'opérateur comme ayant une coloration positive.

11. Procédé selon la revendication 5, comprenant l'étape consistant à compter automatiquement les cellules sélectionnées pour être affichées comme ayant un intérêt diagnostique.

12. Procédé selon la revendication 5, comprenant l'étape consistant à compter automatiquement les cellules sélectionnées pour être affichées comme ayant une coloration positive.

13. Procédé selon la revendication 1, dans lequel ledit marqueur immunochimique est un colorant MiB-1.

14. Procédé selon la revendication 1, dans lequel ledit marqueur immunochimique colore les noyaux des cellules proliférantes.

15. Procédé selon la revendication 14, comprenant l'étape consistant à calculer la zone desdits noyaux colorés.

16. Procédé selon la revendication 1, dans lequel lesdites cellules sont un prélèvement histologique.

17. Procédé de classification d'un prélèvement sur la présence de cellules prémalignes ou malignes, comprenant les étapes consistant à :
a) traiter un prélèvement avec un marqueur immunochimique pour obtenir une indication visuelle de la prolifération cellulaire ou de tout autre processus biologique ;
b) classer des objets individuels dans le prélèvement selon un ordre en fonction de la probabilité pour chaque objet de posséder des attributs en rapport avec une cellule ou un agrégat de cellules prémalignes ou malignes ;
c) sélectionner un ensemble desdits objets conformément audit classement ; et
d) déterminer un index quantitatif d'un colorant immunochimique du prélèvement.

18. Procédé selon la revendication 17, ladite détermination consistant à déterminer un tel index quantitatif pour une corrélation clinique comme un indicateur pronostique.

19. Procédé selon la revendication 17, comprenant la sélection du prélèvement comme un prélèvement cytologique ou histologique.

20. Procédé de classification de prélèvements cytologiques ou histologiques, comprenant les étapes consistant à :
a) traiter un prélèvement avec un marqueur immunochimique pour obtenir une indication visuelle de la prolifération cellulaire ou de tout autre processus biologique ;
b) classer des objets individuels dans le prélèvement selon un ordre en fonction de la probabilité pour chaque objet de posséder des attributs en rapport avec un intérêt spécifié ;
c) sélectionner un ensemble desdits objets conformément audit classement ; et
d) déterminer un index quantitatif d'un colorant immunochimique du prélèvement.
